# EUROPEAN PATENT SPECIFICATION

(11) **EP 0 816 337 B1**
(45) Date of publication and mention of the grant of the patent: **08.08.2001**
(21) Application number: 97304480.3
(22) Date of filing: 25.06.1997
(51) Int. Cl.: C07D 207/34, C07D 207/42, C07D 207/32, C07C 255/44

(54) **A process for the manufacture of 2 aryl 5 perfluoroalkylpyrrole derivatives and intermediates useful therefor**
Verfahren zur Herstellung von 2-Aryl-5-perfluoralkylpyrrol-Derivaten und Zwischenprodukte für dieses Verfahren
Procédé pour la préparation de dérivés de 2-aryl-5-perfluoroalkylpyrrole et intermédiaires pour ce procédé

(30) Priority: 28.06.1996 US 675504
(43) Date of publication of application: 07.01.1998
(73) Proprietor: BASF AKTIENGESELLSCHAFT, 67056 Ludwigshafen (DE)
(72) Inventor: Kameswaran, Venkataraman, Princeton Junction, New Jersey 08550 (US)
(74) Representative: Langfinger, Klaus Dieter, Dr.

(56) References cited:
- EP-A- 0 492 093
- EP-A- 0 530 147
- EP-A- 0 662 475
- US-A- 5 030 735

## Description

Arylpyrrole carbonitrile compounds are highly effective insecticidal, acaricidal and nematocidal agents with a unique mode of action and a broad spectrum of activity. In particular, 2-aryl-5-(trifluoromethyl)pyrrole-3-carbonitrile compounds demonstrate effective control across a wide array of pests and can control resistant pests such as pyrethroid-, organophosphate-, cyclodiene-, organochlorine-, organotin-, carbamate-, and benzophenyl-urea-resistant biotypes of *Helicoverpa/Heliothis* spp., *Spodoptera* spp., *Trichoplusia* spp., *Pseudoplusia* spp. and *Tetranychus* spp.. Because there is no apparent cross-resistance, 2-aryl-5-(trifluoromethyl)pyrrole-3-carbonitrile compounds and their derivatives have potential for use in resistance management programs. Further, said pyrroles have little effect on beneficial species making them excellent candidates for integrated pest management programs, as well. These programs are essential in'today's crop production.

Therefore, methods to prepare said pyrroles and intermediates to facilitate their manufacture are of great use. Among the present methods to prepare 2-aryl-5-(trifluoromethyl)pyrrole-3-carbonitrile on a manufacturing scale are the 1,3-dipolar cycloaddition of 3-oxazolin-5-one with 2-chloroacrylonitrile (U.S. 5,030,735) and the cycloaddition reaction of the appropriate oxazole amine derivatives with 2-chloro-acrylonitrile or 2,3-dichloropropionitrile (U.S. 5,446,170).

Also known is the 1,3-dipolar cycloaddition of the mesionic intermediate product of the acid catalyzed cyclization of a Reissert compound with a suitable alkyne to give an N-substituted pyrrole product as described by W. M. McEwen, *et al*, Journal of Organic Chemistry, 1980, 45, 1301-1308. However, these mesionic intermediates undergo a 1,4-cycloaddition reaction with ethylenic dieneophiles to give an aroylpyrole derivative and, as such, are not useful insecticidal arylpyrrole precursors.

Other processes are described in: EP 662 475, which is directed to the preparation of 3-oxazoline-5-ones; EP 492 093, in which 2-arylamino acids are reacted with a 1,3,-dieneophile in the presence of an organic base and an acid anhydride; EP 530 147 which discloses 3-cyano-2-phenyl-pyrroles and methods for their manufacture.

Therefore, it is an object of this invention to provide an alternative and efficient process of manufacture for the arylpyrrole class of highly effective pesticides.

It is a another object of this invention to provide a source of important intermediate amide nitrile compounds useful in the manufacture of arylpyrrole pesticidal agents.

It is an advantage of this invention that the manufacturing process consists of a single effective step which produces a formula I arylpyrrole precursor capable of being converted to a wide variety of highly effective insecticidal, acaricidal and nematocidal agents.

It is a further advantage of this invention that the overall number of synthetic steps from the initial Strecker reaction product to the final insecticidal arylpyrrole product is reduced from the number of synthetic steps required by employing current known methods such as those described hereinabove.

It is a feature of this invention that the process provides a regiospecific product. These and other features and objects of the invention will become more apparent from the detailed description set forth hereinbelow.

The present invention provides a single step effective process for the manufacture of a 2-aryl-5-perfluoroalkylpyrrole of formula I 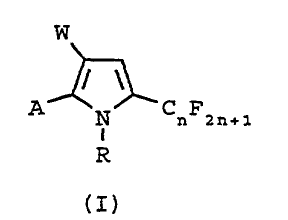
wherein R is hydrogen or C₁-C₆alkyl optionally substituted with one C₁-C₄alkoxy or phenyl group;
n is an integer of 1, 2, 3, 4, 5, 6, 7 or 8;
W is CN, NO₂, COOR₁, or COR₂; 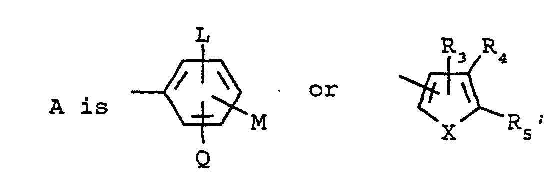
L is hydrogen or halogen;
M and Q are each independently hydrogen, halogen, CN, NO₂, C₁-C₄alkyl, C₁-C₄haloalkyl, C₁-C₄alkoxy, C₁-C₄haloalkoxy, C₁-C₄alkylthio, C₁-C₄alkylsulfinyl or when M and Q are on adjacent positions they may be taken together with the carbon atoms to which they are attached to form a ring in which MQ represents the structure
   -OCH₂O-, -OCF₂O- or -CH=CH-CH=CH-;
R₁ and R₂ are each independently C₁-C₄alkyl;
R₃, R₄, and R₅ are each independently hydrogen, halogen, NO₂, CHO or R₄ and R₅ may be taken together with the atoms to which they are attached to form a ring in which R₄R₅ is represented by the structure 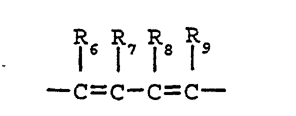
R₆, R₇, R₈ and R₉ are each independently hydrogen, halogen, CN or NO₂; and
X is O or S
which comprises reacting an amide nitrile of formula II 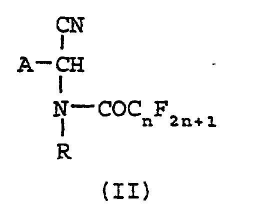 wherein A, R and n are as described hereinabove for formula I with at least one molar equivalent of a dieneophile of formula III 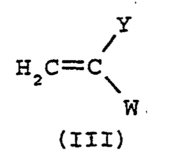 wherein W is as described hereinabove for formula I and Y is hydrogen, Br or Cl with the proviso that when R is hydrogen, then Y must be Br or Cl, in the presence of an acid and a solvent and essentially in the absence of consequences of water. To the extent water is present in the process of the invention, hydrolysis of the starting materials will take place resulting in lower yields and decreased purity of the desired arylpyrrole end product.

The term halogen as used in the specification and claims designates Cl, Br, F or I and the term haloalkyl embraces any alkyl group of χ carbon atoms which may contain from 1 to 2χ+1 halogen atoms which may be the same or different.

The present invention also provides an amide nitrile compound of formula IIa 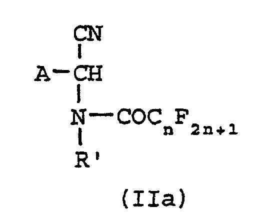 wherein n and A are as described hereinabove for formula I and R' is C₁-C₆alkyl optionally substituted with one C₁-C₄alkoxy or phenyl group.

Processes, to be useful on a manufacturing scale, preferentially contain key intermediate compounds which may be obtained in high to quantitative yield, which are stable either upon isolation or *in situ*, which may be produced from simple or readily available starting materials and which may be readily converted to the desired end-product of manufacture in a minimum of reaction steps, in optimum yield and purity and, if applicable, regio- or stereospecifically.

It has now been found that 2-aryl-5-perfluoroalkylpyrroles of formula I may be prepared directly from the perfluoroacylated amino nitrile product of formula II via a single step synthesis, by the reaction of the formula II compound with a dieneophile of formula III in the presence of an acid and a solvent and essentially in the absence of water. The reaction is shown in flow Diagram I wherein n, A, R and W are as defined hereinabove.

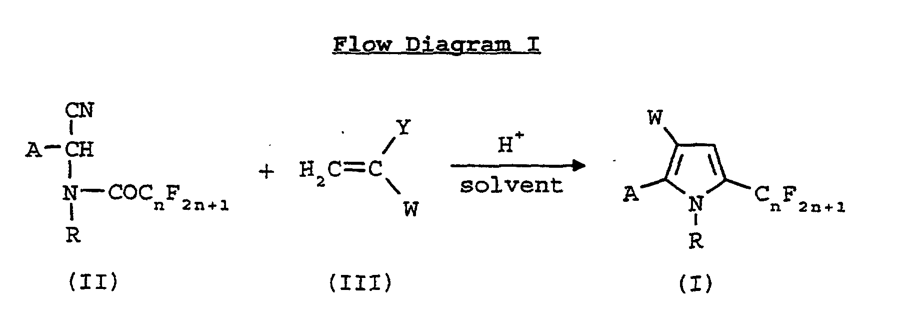

Solvents contemplated for use in the process of the invention are those solvents capable of sustaining essentially anhydrous conditions and partial or complete dissolution of the amide nitrile compound of formula II. Said solvents include organic solvents such as: aromatic hydrocarbons such as benzene, xylene, toluene and the like, preferably toluene; chlorinated aromatic hydrocarbons such as chlorobenzene; carboxylic acid amides such as dimethylformamide, N-methylpyrrolidone, and the like, preferably dimethylformamide; nitriles such as acetonitrile, propionitrile, and the like; alcohols such as isopropanol, *t*-butanol, *sec*-butanol, and the like, preferably t-butanol. These solvents may be used alone or in combination of two or more.

Acids suitable for use in the process of the invention are any acids capable of relative dehydration since increased water gives decreased yield or purity. Among the suitable acids are sulfuric acid, methanesulfonic acid, trifluoromethanesulfonic acid, *p*-toluenesulfonic acid, tetrafluoroboric acid, tetrafluoroboric acid complexes, and the like. Boron trifluoride complexes such as borontrifluoride acetic acid, boron trifluoride dihydrate, and the like are also suitable acids.

Preferred arylpyrrole compounds of formula I obtained by the process of the invention are those wherein n is 1 or 2, W is CN and A is optionally substituted phenyl. More preferred formula I compounds obtained by the inventive process are those wherein n is 1 or 2, W is CN, A is optionally substituted phenyl and R is hydrogen, methyl or ethoxymethyl; particularly preferred are those wherein n is 1, W is CN, A is *p*-chlorophenyl, 2,5-dichlorophenyl, 3,4,5-trichlorophenyl, *p*-bromophenyl, *α,α,α*-trifluoro-*p*-tolyl or *p*-trifluoromethoxyphenyl and R is hydrogen, methyl or ethoxymethyl.

The formula II amide nitrile compounds wherein R is hydrogen and their preparation are described in U.S. 5,426,225. Formula II amide nitrile compounds wherein R is other than hydrogen, i.e. formula IIa, may be obtained via the perfluoroacylation of the appropriate amino nitrile of formula VI. The formula VI aminonitriles are correspondingly readily obtained from their available benzaldehyde, furfurylaldehyde or thienylmethylaldehyde precursors via the well-known Strecker reaction. The reaction sequence is shown in Flow Diagram II wherein n, A and R are as described hereinabove for formula I, m is an integer of 1 or 2, X₁ is Cl, OR₁₀ or O and R₁₀ is hydrogen or C₁-C₆alkyl with the proviso that when X₁ is O, then m must be 2 and when X₁ is Cl or OR₁₀, then m must be 1.

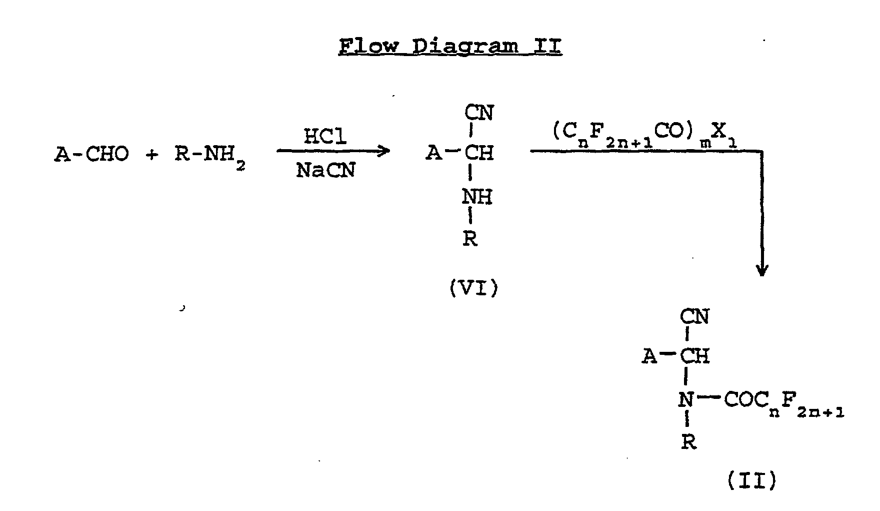

Advantageously, the amide nitrile of formula II may be converted directly to the desired 2-aryl-5-perfluoro-alkylpyrrole of formula I in a single step as shown hereinabove in flow diagram I.

In accordance with the inventive process, the amide nitrile of formula II may be dispersed in a suitable solvent or solvent mixture and admixed with at least one molar equivalent of an appropriate dieneophile of formula III in the presence of an acid under essentially anhydrous reaction conditions. The thus-obtained formula I pyrrole may be isolated using conventional means such as extraction, filtration, distillation, chromatographic separation or the like. The rate of formation of the formula I pyrrole may be increased with increased temperature. However, it is understood that excessively high reaction temperatures may cause decomposition or undesired side reactions and a concomitant decrease in product yield and purity. Typical reaction temperatures may range from 20°C to the reflux temperature of the solvent or solvent mixture. Typically, temperatures of about 20°-150°C are employed, preferably 40°-100°C.

The present invention also provides amide nitrile intermediates of formula IIa 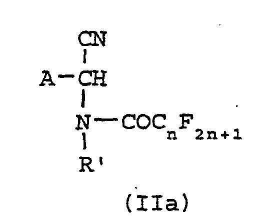
wherein n is an integer of 1, 2, 3, 4, 5, 6, 7 or 8;
R' is C₁-C₆alkyl optionally substituted with one C₁-C₄alkoxy or phenyl group; 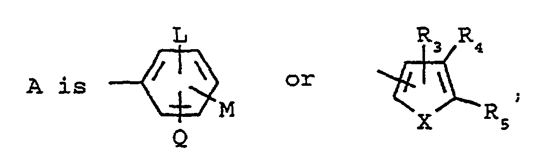
L is hydrogen or halogen;
M and Q are each independently hydrogen, halogen, CN, NO₂, C₁-C₄alkyl, C₁-C₄haloalkyl, C₁-C₄alkoxy, C₁-C₄haloalkoxy, C₁-C₄alkylthio, C₁-C₄alkylsulfinyl or when M and Q are on adjacent positions they may be taken together with the carbon atoms to which they are attached to form a ring in which MQ represents the structure
   -OCH₂O-, -OCF₂O- or -CH=CH-CH=CH-;
R₁ and R₂ are each independently C₁-C₄alkyl;
R₃, R₄, and R₅ are each independently hydrogen, halogen, NO₂, CHO or R₄ and R₅ may be taken together with the atoms to which they are attached to form a ring in which R₄R₅ is represented by the structure 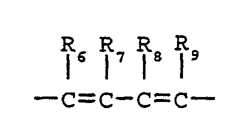
R₆, R₇, R₈ and R₉ are each independently hydrogen, halogen, CN or NO₂; and
X is O or S.

Preferred formula IIa intermediate compounds of the invention are those wherein n is 1 or 2, R' is methyl or ethoxymethyl and A is optionally substituted phenyl.

More preferred compounds of formula IIa are those wherein n is 1, R' is methyl and A is *p*-chlorophenyl, *p*-bromophenyl, 3,5-dichlorophenyl, 3,4,5-trichlorophenyl, *p*-(α,α,α-trifluoro)tolyl or p-trifluoromethoxy phenyl.

While the formula I compounds have insecticidal activity, their greatest utility may be as precursors to certain formula IV compounds. Advantageously, the process of the invention allows the preparation of formula IV 2-aryl-4-halo-5-(perfluoroalkyl)pyrrole-3-carbonitrile insecticidal, acaricidal and nematocidal agents in as few as four synthetic steps from readily available arylaldehyde starting materials. Thus, perfluoroacylation of the Strecker reaction product (VI) followed by the inventive process step gives the arylpyrrole precursor (I), which may be halogenated to give the desired pesticidal product (IV). The synthesis is illustrated in flow diagram III, wherein n, A, R, Y and W are as described hereinabove and Hal is halogen, preferably Br or Cl.

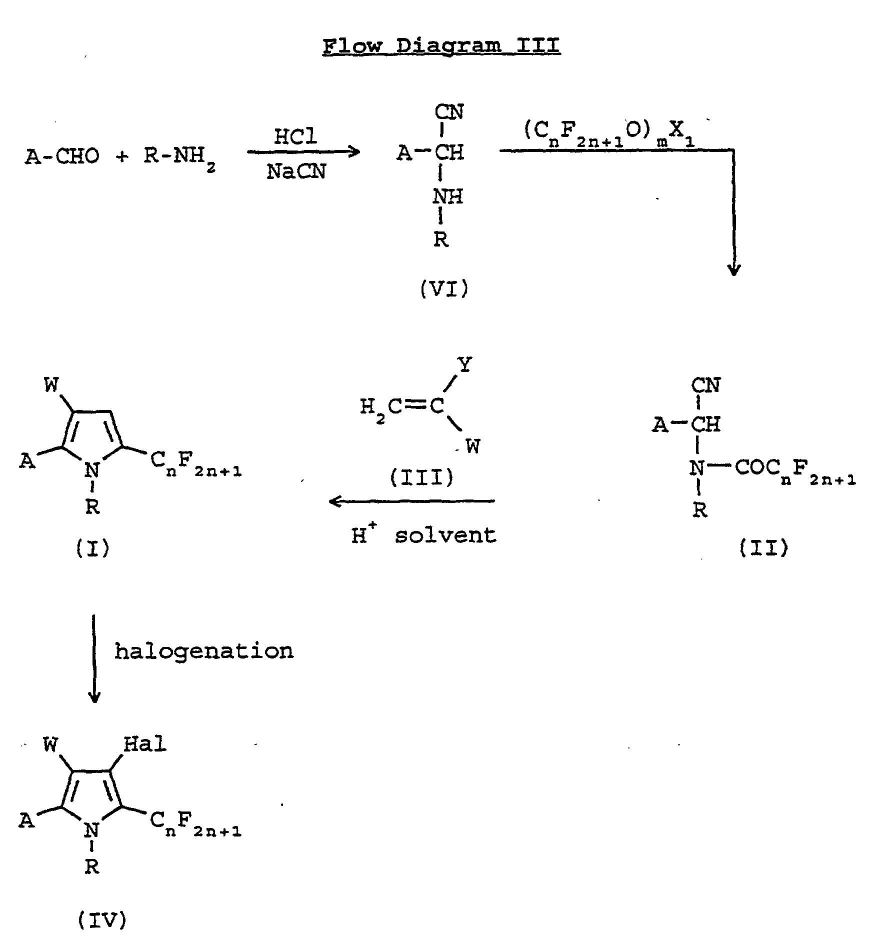

Halogenation methods may be any known methods such as those described in U.S. 5,010,098 or U.S. 5,449,789.

In order to provide a more clear understanding of the invention, the following examples are set forth below. These examples are merely illustrative and are not to be understood to limit the scope or underlying principles of the invention in any way. Indeed, various modifications of the invention, in addition to those shown and describe d herein, will become apparent to those skilled in the art from the following examples and the foregoing description. Such modification s are also intended to fall within the scope of the appended claims.

The terms ¹H NMR, ¹³C NMR and ¹⁹F NMR designate proton, carbon 13 and fluorine 19 nuclear magnetic resonance, respectively. The term HPLC designates high performance liquid chromatography.

### EXAMPLE 1

### Preparation of N-Isopropylamino(p-chlorophenyl)acetonitrile

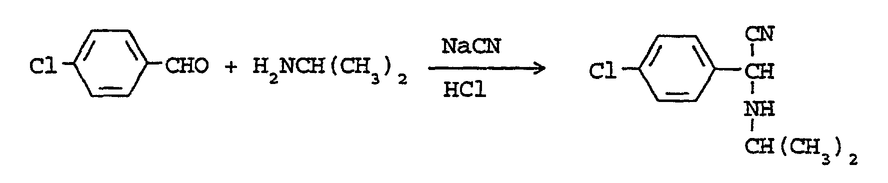

Isopropylamine (88.7g, 1.5 mol) is added to an aqueous solution of concentrated hydrochloric acid (125 mL, 1.5 mL) in water at 25°-30°C. The resultant mixture is treated sequentially with a solution of sodium cyanide (53.9g, 1.1 mol) in water and methylene chloride at 30°C, warmed to 35°C, treated with a solution of *p*-chlorobenzaldehyde (140.6g, 1 mol) in methylene chloride over a 15-25 minute period, allowed to warm, held for 3 hours at 45°C and cooled to room temperature. The phases are separated and the organic phase is washed with water and concentrated *in vacuo* to give a residue. The residue is crystallized from heptane to give the title product as a pale yellow crystalline solid, 190.3g (91.2% yield), mp 72.0°-73.0°C, identified by ¹H and ¹³C NMR analyses.

### EXAMPLE 2

### Preparation of N-(p-chloro-α-cyanobenzyl)-2,2,2-trifluoro-N-isopropylacetamide

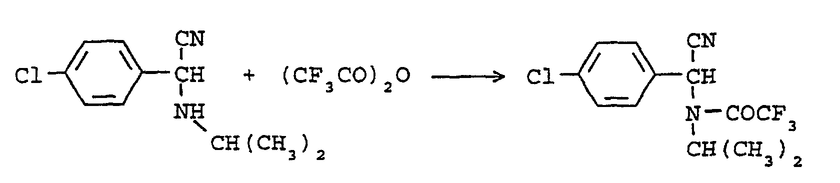

A slurry of N-isopropylamino(p-chlorophenyl)acetonitrile (25.0g, 0.12 mol) in trifluoroacetic anhydride is gently heated at reflux temperature for 20 hours and concentrated *in vacuo* to give an oil residue. The oil is crystallized from toluene/heptane to give the title product as a white solid, 26.5g (72.4% yield) mp 78.5°-79.5°C, identified by ¹H, ¹³C and ¹⁹F NMR analyses.

### EXAMPLE 3

### Preparation of N-Benzyl-N-(p-chloro-α-cyanobenzyl)-2,2,2-trifluoroacetamide

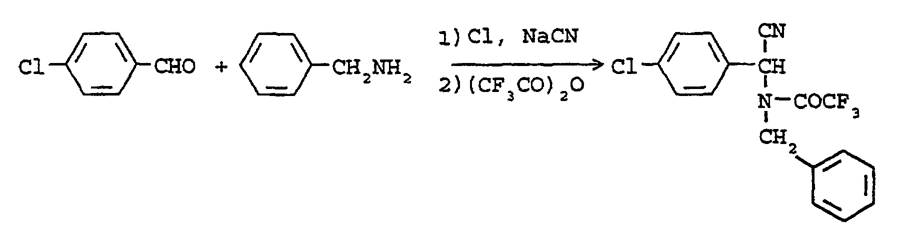

Aqueous hydrochloric acid (62.5 mL of 12 N, 0.75 mol) in water (100 mL) is treated with benzylamine (80.4g, 0.75 mol) at <20°C, then treated sequentially with a solution of sodium cyanide (27.0g, 0.55 mol) in water and methylene chloride, warmed to 35°C, treated with a solution of p-chlorobenzaldehyde (70.3g, 0.5 mol) in methylene chloride, allowed to warm to 50°C, and held at 45°C for 3.5 hours. The phases are separated and the organic phase is washed with water and concentrated to a syrup residue. The residue is dissolved in toluene and ethyl acetate, treated with trifluoroacetic anhydride (105.0g, 0.5 mol) at 20°-30°C over a 30 minute period and diluted with heptane. The resultant white fluffy solid precipitate is filtered and dried to give the title product, 119.8g (70.7% yield), mp 131°-132°C, identified by ¹H, ¹³C and ¹⁹F NMR analyses.

### EXAMPLE 4

### Preparation of 2-(p-Chlorophenyl)-5-(trifluoromethyl)-pyrrole-3-carbonitrile

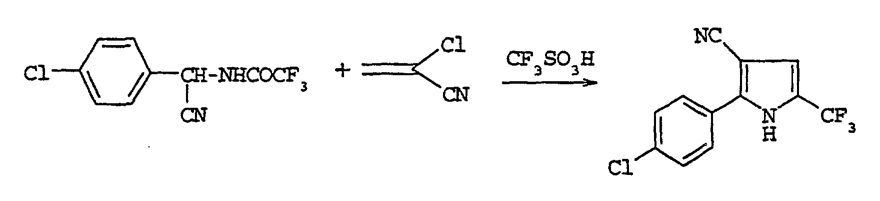

A mixture of N-(*p*-chloro-α-cyanobenzyl) -2,2,2-trifluoroacetamide (10.5g, 0.04 mol) and toluene is cooled to 5°-10°C under a nitrogen atmosphere, treated with trifluoromethanesulfonic acid (12.0g, 0.08 mol) over a 20 minute period, allowed to warm to room temperature and held at 25°C for 3 hours. The formation of the intermediate 5-amino oxazole salt is monitored by ¹⁹F NMR (DMSO-d₆). When the intermediate salt formation is complete, the mixture is cooled below 20°C, treated with dimethylformamide and 2-chloroacrylonitrile (5.25g, 0.06 mol), held at 25°C for 16-18 hours, and treated with ethyl acetate and water. The phases are separated and the organic phase is washed with water and concentrated *in* *vacuo* to give a solid residue. Flash column chromatography of the residue on silica gel, packed and eluted respectively with 15% and 20% ethyl acetate in heptane gives the title product as pale yellow crystals, 6.14g (57% yield), mp 237°-240°C, identified by HPLC and NMR analyses.

### EXAMPLE 5

### Preparation of 2-Aryl-5-(trifluoromethyl)pyrrole derivatives

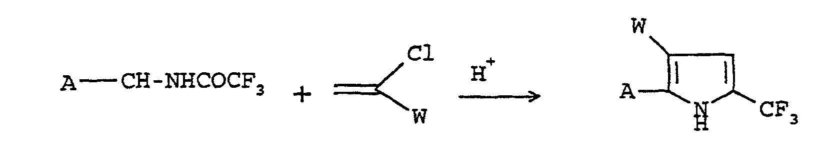

Using essentially the same procedure described in Example 4 and substituting the appropriate trifluoro-acetylated amino nitrile starting material, the following products are obtained:

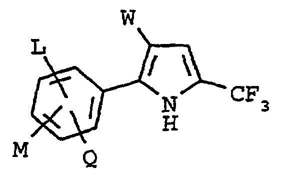

| **W** | **L** | **M** | **O** | **mp °C** | **% yield** |
|---|---|---|---|---|---|
| CN | 3-Cl | 4-Cl | H | 241-244 | 55 |
| CN | H | 4-Br | H | 249-251 | 35 |

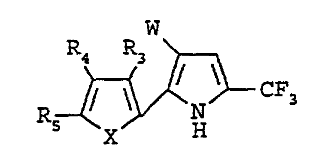

| **W** | **X** | **R**_{**3**} | **R**_{**4**} | **R**_{**5**} | **mp °C** | **% yield** |
|---|---|---|---|---|---|---|
| CN | S | H | H | H | | |

### EXAMPLE 6

### Preparation of 2-(p-Chlorophenyl)-5-trifluoromethyl)-pyrrole-3-carbonitrile

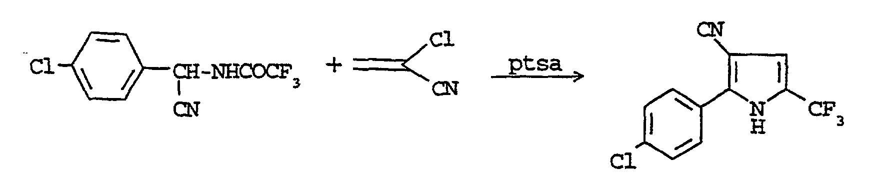

A mixture of *p*-toluenesulfonic acid (ptsa), monohydrate (19.1g, 0.1 mol) in toluene is azeotropically dried using a Dean-Stark trap to obtain the anhydrous acid. Toluene is then removed *in vacuo* and replaced with propionitrile. The resultant solution is treated with N-(*p*-chloro-α-cyanobenzyl)-2,2,2-trifluoroacetamide (13.1g, 0.05 mol) and 2-chloroacrylonitrile (8.75g, 0.1 mol), heated at 98°-100°C for 18 hours, cooled to room temperature and quenched with a mixture of water and ethyl acetate. The phases are separated and the organic phase is washed with water and concentrated *in vacuo* to give a residue. Flash column chromatography of the residue using silica gel packed and eluted with 20% ethyl acetate in heptane gives the title product as pale yellow crystals, 4.2g (39% yield), identified by HPLC and NMR analyses.

### EXAMPLE 7

### Preparation of Methyl 2-(p-chlorophenyl)-5-(trifluoromethyl)pyrrole-3-carboxylate

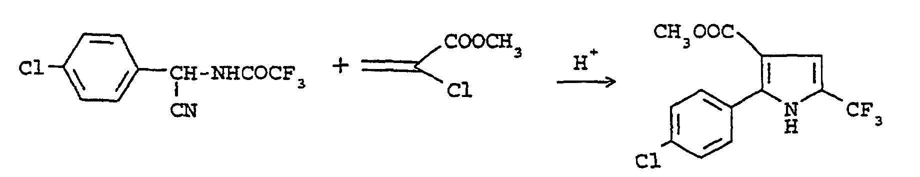

A slurry of N-(*p*-chloro-α-cyanobenzyl)-2,2,2-trifluoroacetamide (10.5g, 0.04 mol) in toluene under a nitrogen atmosphere is cooled to 10°C, treated with trifluoromethanesulfonic acid (12.0g, 0.08 mol) over 10-15 minutes, allowed to warm to room temperature and stirred for 3 hours. The reaction is monitored by ¹⁹F NMR (DMSO-d₆) analysis to show completion of the intermediate salt formation. When formation is complete, the mixture is cooled to 10°C, treated with dimethylformamide, treated with methyl 2-chloroacrylate (7.2g, 0.06 mol), allowed to warm to room temperature, stirred for 18 hours, and diluted with water and ethyl acetate. The phases are separated and the organic phase is washed with water and concentrated to give a waxy solid residue. Flash column chromatography of the residue on silica gel, packed and eluted with 20% ethyl acetate in heptane gives the title product as a white crystalline solid, 7.6g (62% yield), mp 123°-125°C identified by ¹H and ¹⁹F NMR analyses.

### Example 8

### Preparation of 2-(p-Chlorophenyl)-1-methyl-5-(trifluoromethyl)pyrrole-3-carbonitrile

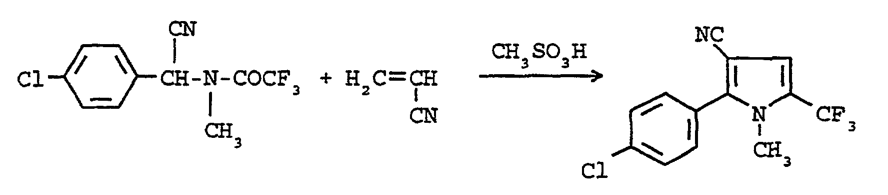

A solution of N-(*p*-chloro-α-cyanobenzyl-2,2,2-trifluoro-N-methylacetamide (13.8g, 0.05 mol) in toluene is treated with acrylonitrile (5.3g, 0.1 mol) and methanesulfonic acid (9.6g, 0.1 mol), heated at 108°-110°C for 30 hours, quenched with water and extracted with ethyl acetate. The organic extracts are combined and concentrated *in vacuo* to give a residue. The residue is purified by flash column chromatography on silica gel, packed and eluted with 15% ethyl acetate in heptane to give the title product as a pale yellow solid, 0.9g (63% yield), mp 129°-130°C, identified by ¹H and ¹⁹F NMR analyses.

### EXAMPLE 9

### Preparation of 2-(p-Chlorophenyl)-1-methyl-5-(trifluoromethyl)pyrrole-3-carbonitrile

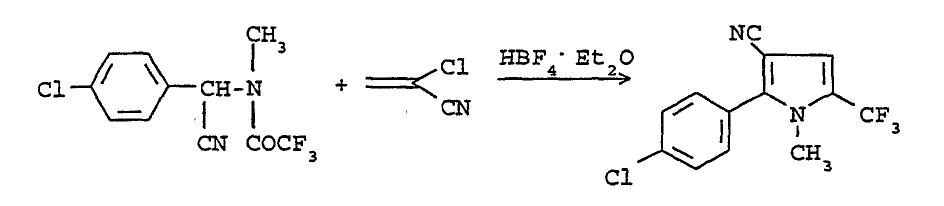

A solution of N-(*p*-chloro-α-cyanobenzyl)-2,2,2-trifluoro-N-methylacetamide (13.8g, 0.05 mol) in toluene is treated with tetrafluoroboric acid-diethyl etherate (10.5g as is, 8.9g real, 0.055 mol) at room temperature, heated to 60°C, treated with 2-chloroacrylonitrile (6.9g, 0.075 mol) over a 25 minute period, held for 2 to 2.5 hours at 60°C, cooled and treated with ethyl acetate. The resultant solution is washed with water and concentrated to give a waxy residue. Flash column chromatography of the residue on silica gel packed with 15% ethyl acetate in heptane and eluted with 20% ethyl acetate in heptane gives the title product as pale yellow crystals, 3.1g (22% yield), mp 129°-130°C, identified by ¹H, ¹³C, and ¹⁹F NMR analyses.

### EXAMPLE 10

### Preparation of 2-(p-Chlorophenyl-1-methyl-5-(trifluoromethyl)pyrrole-3-carbonitrile

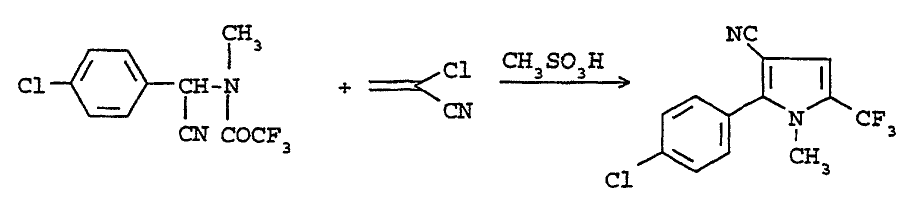

A solution of N-(*p*-chloro-α-cyanobenzyl)-2,2,2-trifluoro-N-methylacetamide (13.8g, 0.05 mol) in toluene is treated with 2-chloroacrylonitrile (4.4g, 0.05 mol) and methanesulfonic acid (4.8g, 0.05 mol), heated at 110°C for 4 hours, treated with a second portion of 2-chloroacrylonitrile (4.4g, 0.05 mol) and methanesulfonic acid (4.8g, 0.05 mol), heated at 110°C for 12 hours, cooled and treated with ethyl acetate and water. The phases are separated and the organic phase is washed with water and concentrated to give a residue. The residue is chromatographed using silica gel packed with 15% ethyl acetate in heptane and eluted with 20% ethyl acetate in heptane to give the title product as pale yellow crystals, 4.8g (34% yield), mp 129°-130°C.

### EXAMPLE 11

### Preparation of 2-(p-Chlorophenyl)-1-isopropyl-5-(trifluoromethyl)pyrrole-3-carbonitrile

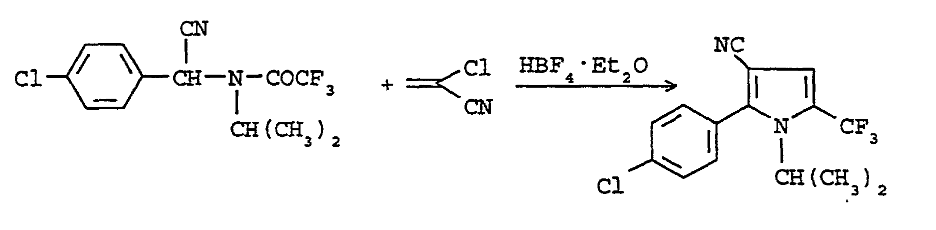

A solution of N-(*p*-chloro-α-cyanobenzyl)-2,2,2-trifluoro-N-isopropylacetamide (6.1g, 0.02 mol) in toluene is treated at room temperature with tetrafluoroboric acid diethyl etherate (4.2g as is, 3.6g real, 0.022 mol) under a nitrogen atmosphere, heated to 60°C, treated with 2-chloroacrylonitrile (2.62g, 0.03 mol) over 15-20 minutes, held at 60°C for 3 hours, cooled to room temperature and treated with ethyl acetate and water. The phases are separated and the organic phase is washed with water and concentrated to give a brown gum residue. Flash column chromatography of the residue on silica gel packed and eluted with 15% ethyl acetate in heptane gives the title product as a brown oil, 1.3g (20.8% yield), identified by ¹H and ¹⁹F NMR and mass spectral analyses.

### EXAMPLE 12

### Preparation of 1-Benzyl-2-(p-chlorophenyl)-5-(trifluoromethyl)pyrrole-3-carbonitrile

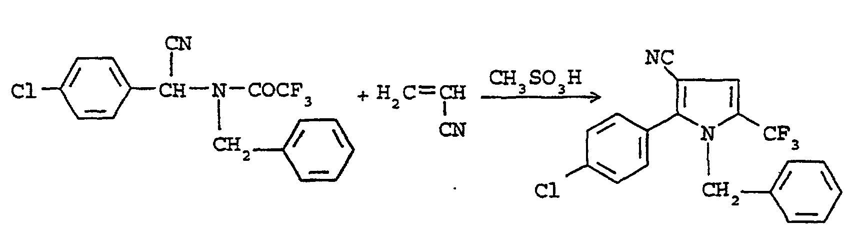

A solution of N-(*p*-chloro-α-cyanobenzyl)-2,2,2-trifluoro-N-benzylacetamide (10.6g, 0.03 mol) in toluene is treated with methanesulfonic acid (3.2g, 0.033 mol) and acrylonitrile (3.9g, 0.045 mol), heated at 100°-105°C for 18 hours, cooled, treated with additional methanesulfonic acid (1.6g, 0.017 mol), heated at 100°-105°C for 22 hours, quenched with water and extracted with ethyl acetate. The organic extracts are combined and concentrated *in vacuo* to give a residue. The residue is purified by flash column chromatography using silica gel packed and eluted with 15% ethyl acetate in heptane to give the title product as white crystals, 4.4g (40.6% yield), mp 103.5°-105.5°C, identified by ¹H, ¹³C and ¹⁹F NMR and mass spectral analyses.

## Claims

1. A process for the manufacture of a compound of formula I 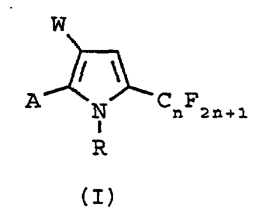
wherein R is hydrogen or C₁-C₆alkyl optionally substituted with one C₁-C₄alkoxy or phenyl group;
n is an integer of 1, 2, 3, 4, 5, 6, 7 or 8;
W is CN, NO₂, COOR₁, or COR₂; 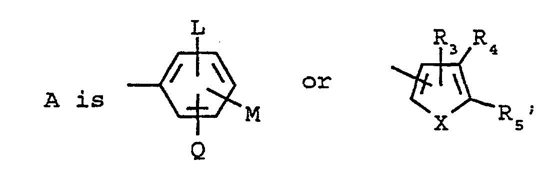
L is hydrogen or halogen;
M and Q are each independently hydrogen, halogen, CN, NO₂, C₁-C₄alkyl, C₁-C₄haloalkyl, C₁-C₄alkoxy, C₁-C₄haloalkoxy, C₁-C₄alkylthio, C₁-C₄alkylsulfinyl or when M and Q are on adjacent positions they may be taken together with the carbon atoms to which they are attached to form a ring in which MQ represents the structure
-OCH₂O-, -OCF₂O- or -CH=CH-CH=CH-;
R₁ and R₂ are each independently C₁-C₄alkyl;
R₃, R₄, and R₅ are each independently hydrogen, halogen, NO₂, CHO or R₄ and R₅ may be taken together with the atoms to which they are attached to form a ring in which R₄R₅ is represented by the structure 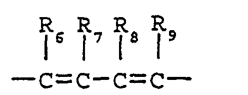
R₆, R₇, R₈ and R₉ are each independently hydrogen, halogen, CN or NO₂ ; and
X is O or S
which comprises reacting an amide nitrile of formula II 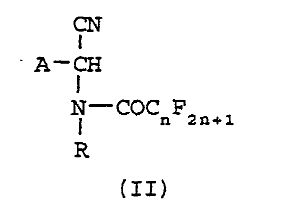 wherein A, R and n are as described hereinabove for formula I with at least one molar equivalent of a dieneophile of formula III 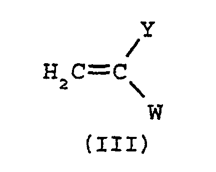 wherein W is as described hereinabove for formula I and Y is hydrogen, Br or Cl with the proviso that when R is hydrogen, then Y must be Br or Cl, in the presence of an acid and a solvent and essentially in the absence of water.

2. The process according to claim 1 wherein the acid is selected from the group consisting of methanesulfonic acid, trifluoromethanesulfonic acid, benzenesulfonic acid, *p*-toluenesulfonic acid, naphthalenesulfonic acid, tetrafluoroboric acid, tetrafluoroboric acid etherate and tetrafluoroboric acid alkanolate.

3. The process according to claim 1 wherein the solvent is an aromatic hydrocarbon, a halogenated aromatic hydrocarbon, an organic amide, a nitrile, an alkanol, or mixtures thereof.

4. The process according to claim 3 wherein the solvent is selected from the group consisting of toluene, dimethylformamide, acetonitrile, propionitrile, *t*-butanol, and mixtures thereof.

5. The process according to claim 1 wherein A is 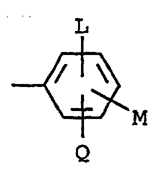 wherein L is hydrogen or halogen; M and Q are each independently hydrogen, halogen, C₁-C₄haloalkyl or C₁-C₄haloalkoxy; n is 1 or 2; W is CN and R is hydrogen or methyl.

6. A process for making a compound of formula IV 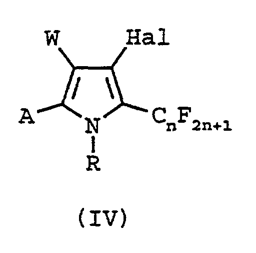
wherein R is hydrogen or C₁-C₆alkyl optionally substituted with one C₁-C₄alkoxy or phenyl group;
n is an integer of 1, 2, 3, 4, 5, 6, 7 or 8;
W is CN, NO₂, COOR₁, or COR₂; 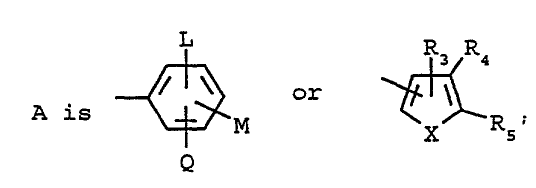
L is hydrogen or halogen;
M and Q are each independently hydrogen, halogen, CN, NO₂, C₁-C₄alkyl, C₁-C₄haloalkyl, C₁-C₄alkoxy, C₁-C₄haloalkoxy, C₁-C₄alkylthio, C₁-C₄alkylsulfinyl or when M and Q are on adjacent positions they may be taken together with the carbon atoms to which they are attached to form a ring in which MQ represents the structure
-OCH₂O-, -OCF₂O- or -CH=CH-CH=CH-;
R₁ and R₂ are each independently C₁-C₄alkyl,
R₃, R₄, and R₅ are each independently hydrogen, halogen, NO₂, CHO or R₄ and R₅ may be taken together with the atoms to which they are attached to form a ring in which R₄R₅ is represented by the structure 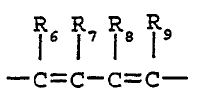
R₆, R₇, R₈ and R₉ are each independently hydrogen, halogen, CN or NO₂;
X is O or S; and
Hal is a halogen atom; which comprises reacting an amide nitrile of formula II 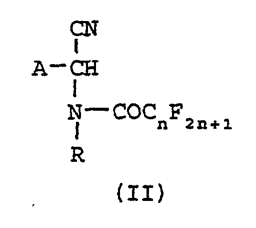 wherein A, R and n are as described hereinabove for formula I with at least one molar equivalent of a dieneophile of formula III 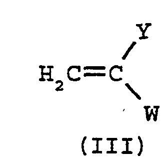 wherein W is as described hereinabove for formula I and Y is hydrogen, Br or Cl with the proviso that when R is hydrogen, then Y must be Br or Cl, in the presence of an acid and a solvent and essentially in the absence of water to form a formula I compound 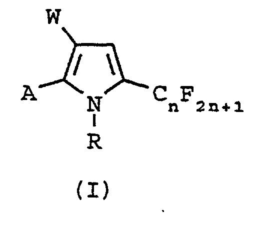 and halogenating said formula I compound to form said formula IV compound.

7. A compound of formula IIa 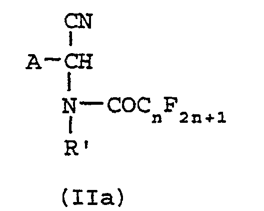
wherein n is an integer of 1, 2, 3, 4, 5, 6, 7 or 8;
R' is C₁-C₆alkyl optionally substituted with one C₁-C₄alkoxy or phenyl group; 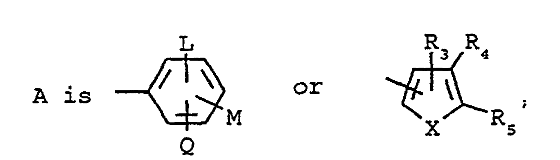
L is hydrogen or halogen;
M and Q are each independently hydrogen, halogen, CN, NO₂, C₁-C₄alkyl, C₁-C₄haloalkyl, C₁-C₄alkoxy, C₁-C₄haloalkoxy, C₁-C₄alkylthio, C₁-C₄alkylsulfinyl or when M and Q are on adjacent positions they may be taken together with the carbon atoms to which they are attached to form a ring in which MQ represents the structure
-OCH₂O-, -OCF₂O- or -CH=CH-CH=CH-;
R₁ and R₂ are each independently C₁-C₄alkyl;
R₃, R₄, and R₅ are each independently hydrogen, halogen, NO₂, CHO or R₄ and R₅ may be taken together with the atoms to which they are attached to form a ring in which R₄R₅ is represented by the structure 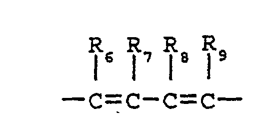
R₆, R₇, R₈ and R₉ are each independently hydrogen, halogen, CN or NO₂; and
X is O or S.

8. The compound according to claim 7 wherein n is 1 or 2.

9. The compound according to claim 7 wherein R' is methyl or ethoxymethyl.

10. The compound according to claim 7 wherein A is 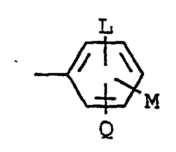 wherein L is hydrogen or halogen and M and Q are each independently hydrogen, halogen or C₁-C₄haloalkyl.

## Patentansprüche

1. Verfahren zur Herstellung einer Verbindung der Formel I 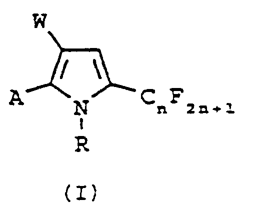 in der R Wasserstoff oder C₁-C₆Alkyl, das gegebenenfalls durch eine C₁-C₄Alkoxy- oder Phenylgruppe substituiert ist, bedeutet,
n eine ganze Zahl 1, 2, 3, 4, 5, 6, 7 oder 8 bedeutet,
W CN, NO₂, COOR₁ oder COR₂ bedeutet, 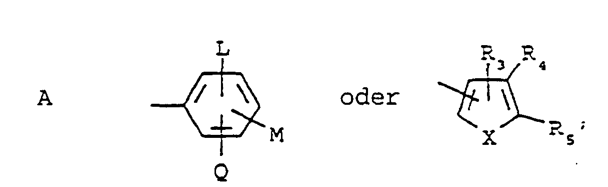 bedeutet,
L Wasserstoff oder Halogen bedeutet,
M und Q jeweils unabhängig voneinander Wasserstoff, Halogen, CN, NO₂, C₁-C₄Alkyl, C₁-C₄Halogenalkyl, C₁-C₄Alkoxy, C₁-C₄Halogenalkoxy, C₁-C₄Alkylthio, C₁-C₄Alkylsulfinyl bedeuten oder, wenn M und Q benachbarte Positionen einnehmen, gemeinsam mit den Kohlenstoffatomen, an die sie gebunden sind, einen Ring bilden können, in dem MQ die Struktur
-OCH₂O-, -OCF₂O- oder -CH=CH-CH=CH-aufweist,
R₁ und R₂ jeweils unabhängig C₁-C₄Alkyl bedeuten,
R₃, R₄ und R₅ jeweils unabhängig Wasserstoff, Halogen, NO₂, CHO bedeuten, oder R₄ und R₅ gemeinsam mit den Atomen, an die sie gebunden sind, einen Ring bilden können, in dem R₄R₅ die Struktur 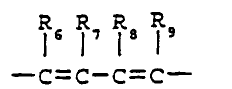 aufweist, R₆, R₇, R₈ und R₉ jeweils unabhängig Wasserstoff, Halogen, CN oder NO₂ bedeuten, und
X O oder S darstellt,
dadurch gekennzeichnet, daß man ein Amidnitril der Formel II 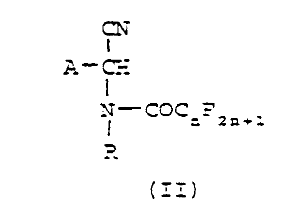 in der A, R und n wie oben für Formel I beschrieben sind, mit mindestens einem Moläquivalent eines Dienophils der Formel III 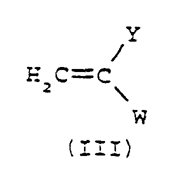 in der W wie oben für Formel I beschrieben ist und Y Wasserstoff, Br oder Cl bedeutet, jedoch unter der Bedingung, daß, wenn R Wasserstoff bedeutet, Y Br oder Cl sein muß, in Gegenwart einer Säure und eines Lösungsmittels und im wesentlichen in Abwesenheit von Wasser umsetzt.

2. Verfahren nach Anspruch 1, wobei die Säure aus der Gruppe Methansulfonsäure, Trifluormethansulfonsäure, Benzolsulfonsäure, p-Toluolsulfonsäure, Naphthalinsulfonsäure, Tetrafluorborsäure, Tetrafluorborsäureetherat und Tetrafluorborsäurealkanolat stammt

3. Verfahren nach Anspruch 1, wobei es sich bei dem Lösungsmittel um einen aromatischen Kohlenwasserstoff, einen halogenierten aromatischen Kohlenwasserstoff, ein organisches Amid, ein Nitril, ein Alkanol oder deren Mischungen handelt.

4. Verfahren nach Anspruch 3, wobei das Lösungsmittel aus der Gruppe Toluol, Dimethylformamid, Acetonitril, Propionitril, t-Butanol und deren Mischungen stammt.

5. Verfahren nach Anspruch 1, wobei A 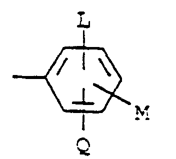 bedeutet,
worin L Wasserstoff oder Halogen bedeutet, M und Q jeweils unabhängig Wasserstoff, Halogen, C₁-C₄Halogenalkyl oder C₁-C₄Halogenalkoxy bedeuten, n 1 oder 2 bedeutet, W CN bedeutet und R Wasserstoff oder Methyl bedeutet.

6. Verfahren zur Herstellung einer Verbindung der Formel IV 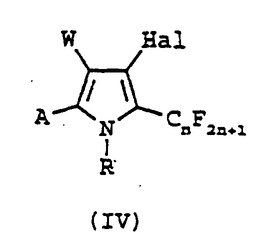 in der R Wasserstoff oder C₁-C₆Alkyl, das gegebenenfalls durch eine C₁-C₄Alkoxy- oder Phenylgruppe substituiert ist, bedeutet,
n eine ganze Zahl 1, 2, 3, 4, 5, 6, 7 oder 8 bedeutet,
W CN, NO₂, COOR₁ oder COR₂ bedeutet, 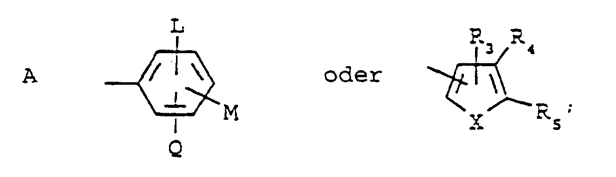 bedeutet,
L Wasserstoff oder Halogen bedeutet,
M und Q jeweils unabhängig voneinander Wasserstoff, Halogen, CN, NO₂, C₁-C₄Alkyl, C₁-C₄Halogenalkyl, C₁-C₄Alkoxy, C₁-C₄Halogenalkoxy, C₁-C₄Alkylthio, C₁-C₄Alkylsulfinyl bedeuten oder, wenn M und Q benachbarte Positionen einnehmen, gemeinsam mit den Kohlenstoffatomen, an die sie gebunden sind, einen Ring bilden können, in dem MQ die Struktur
-OCH₂O-, -OCF₂O- oder -CH=CH-CH=CH-aufweist,
R₁ und R₂ jeweils unabhängig C₁-C₄Alkyl bedeuten,
R₃, R₄ und R₅ jeweils unabhängig Wasserstoff, Halogen, NO₂, CHO bedeuten, oder R₄ und R₅ gemeinsam mit den Atomen, an die sie gebunden sind, einen Ring bilden können, in dem R₄R₅ die Struktur 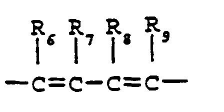 aufweist, R₆, R₇, R₈ und R₉ jeweils unabhängig Wasserstoff, Halogen, CN oder NO₂ bedeuten,
X O oder S bedeutet, und
Hal ein Halogenatom bedeutet, dadurch gekennzeichnet, daß man ein Amidnitril der Formel II 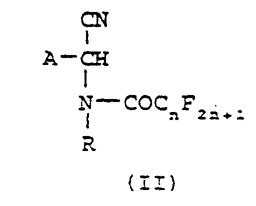 in der A, R und n wie oben für Formel I beschrieben sind, mit mindestens einem Moläquivalent eines Dienophils der Formel III 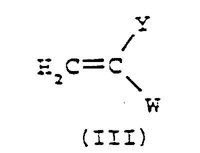 in der W wie oben für Formel I beschrieben ist und Y Wasserstoff, Br oder Cl bedeutet, jedoch unter der Bedingung, daß, wenn R Wasserstoff bedeutet, Y Br oder Cl sein muß, in Gegenwart einer Säure und eines Lösungsmittels und im wesentlichen in Abwesenheit von Wasser unter Bildung einer Verbindung der Formel I 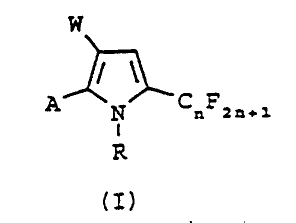 umsetzt und diese Verbindung der Formel I unter Bildung der Verbindung der Formel IV halogeniert.

7. Verbindung der Formel IIa 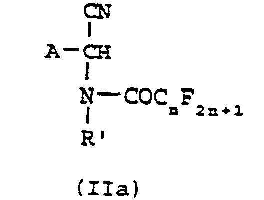 in der n eine ganze Zahl 1, 2, 3, 4, 5, 6, 7 oder 8 bedeutet,
R' C₁-C₆Alkyl, das gegebenenfalls durch eine C₁-C₄Alkoxy- oder Phenylgruppe substituiert ist, bedeutet, 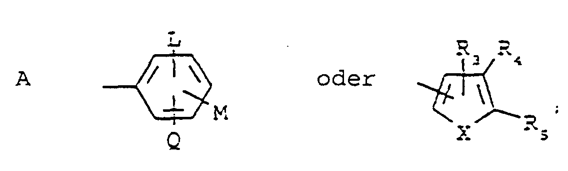 bedeutet,
L Wasserstoff oder Halogen bedeutet,
M und Q jeweils unabhängig voneinander Wasserstoff, Halogen, CN, NO₂, C₁-C₄Alkyl, C₁-C₄Halogenalkyl, C₁-C₄Alkoxy, C₁-C₄Halogenalkoxy, C₁-C₄Alkylthio, C₁-C₄Alkylsulfinyl bedeuten oder, wenn M und Q benachbarte Positionen einnehmen, gemeinsam mit den Kohlenstoffatomen, an die sie gebunden sind, einen Ring bilden können, in dem MQ die Struktur
-OCH₂O-, -OCF₂O- oder -CH=CH-CH=CH-aufweist,
R₁ und R₂ jeweils unabhängig C₁-C₄Alkyl bedeuten,
R₃, R₄ und R₅ jeweils unabhängig Wasserstoff, Halogen, NO₂, CHO bedeuten, oder R₄ und R₅ gemeinsam mit den Atomen, an die sie gebunden sind, einen Ring bilden können, in dem R₄R₅ die Struktur 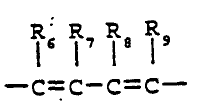 aufweist, R₆, R₇, R₈ und R₉ jeweils unabhängig Wasserstoff, Halogen, CN oder NO₂ bedeuten, und
X O oder S bedeutet

8. Verbindung nach Anspruch 7, wobei n 1 oder 2 bedeutet.

9. Verbindung nach Anspruch 7, wobei R' Methyl oder Ethoxymethyl bedeutet.

10. Verbindung nach Anspruch 7, wobei A 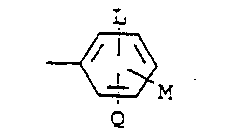 bedeutet, worin L Wasserstoff oder Halogen bedeutet und M und Q jeweils unabhängig Wasserstoff, Halogen oder C₁-C₄Halogenalkyl bedeuten.

## Revendications

1. Procédé pour la fabrication d'un composé de formule I 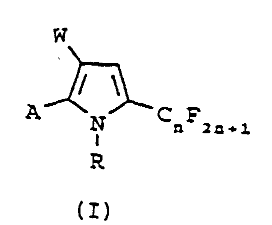 dans laquelle
R est l'hydrogène ou un groupe alkyle en C₁-C₆ facultativement substitué par un groupe alcoxy en C₁-C₄ ou phényle ;
n est un nombre entier 1, 2, 3, 4, 5, 6, 7 ou 8 ;
W est CN, NO₂, COOR₁ ou COR₂ ; 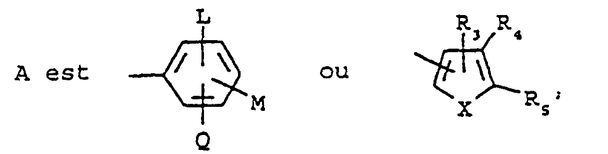
L est l'hydrogène ou un halogène ;
M et Q sont chacun indépendamment l'hydrogène, un halogène, CN, NO₂, un groupe alkyle en C₁-C₄, halogénoalkyle en C₁-C₄, alcoxy en C₁-C₄, halogénoalcoxy en C₁-C₄, alkylthio en C₁-C₄, alkylsulfinyle en C₁-C₄, ou bien, lorsque M et Q sont sur des positions adjacentes, ils peuvent être pris avec les atomes de carbone auxquels ils sont fixés pour former un cycle dans lequel MQ représente la structure
-OCH₂O-, -OCF₂O- ou -CH=CH-CH=CH- ;
R₁ et R₂ sont chacun indépendamment un groupe alkyle en C₁-C₄ ;
R₃, R₄ et R₅ sont chacun indépendamment l'hydrogène, un halogène, NO₂, CHO, ou bien R₄ et R₅ peuvent être pris avec les atomes auxquels ils sont fixés pour former un cycle dans lequel R₄R₅ représente la structure 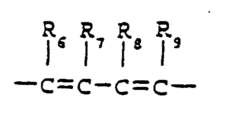
R₆, R₇, R₆ et R₉ sont chacun indépendamment l'hydrogène, un halogène, CN ou NO₂ ; et
X est O ou S,
qui consiste à faire réagir un amide-nitrile de formule II 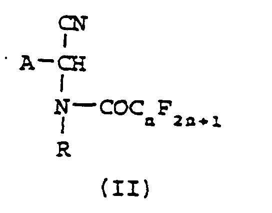 dans laquelle A, R et n sont tels que définis ci-dessus pour la formule I, avec au moins un équivalent molaire d'un composé diénophile de formule III 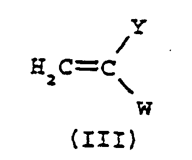 dans laquelle W est tel que défini ci-dessus pour la formule I et Y est l'hydrogène, Br ou Cl, à condition que si R est l'hydrogène, alors Y soit Br ou Cl, en présence d'un acide et d'un solvant et essentiellement en l'absence d'eau.

2. Procédé selon la revendication 1, dans lequel l'acide est choisi dans la classe formée par l'acide méthanesulfonique, l'acide trifluorométhanesulfonique, l'acide benzènesulfonique, l'acide p-toluènesulfonique, l'acide naphtalènesulfonique, l'acide tétrafluoroborique, un complexe éthéré d'acide tétrafluoroborique et un alcanolate d'acide tétrafluoroborique.

3. Procédé selon la revendication 1, dans lequel le solvant est un hydrocarbure aromatique, un hydrocarbure aromatique halogéné, un amide organique, un nitrile, un alcanol ou des mélanges d'entre eux.

4. Procédé selon la revendication 3, dans lequel le solvant est choisi dans la classe formée par le toluène, le diméthylformamide, l'acétonitrile, le propionitrile, le t-butanol et leurs mélanges.

5. Procédé selon la revendication 1, dans lequel A est 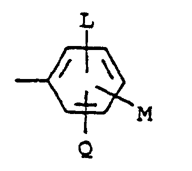 où L est l'hydrogène ou un halogène ; M et Q sont chacun indépendamment l'hydrogène, un halogène, un groupe halogénoalkyle en C₁-C₄ ou halogénoalcoxy en C₁-C₄ ; n est 1 ou 2 ; W est CN et R est l'hydrogène ou un groupe méthyle.

6. Procédé pour la fabrication d'un composé de formule IV 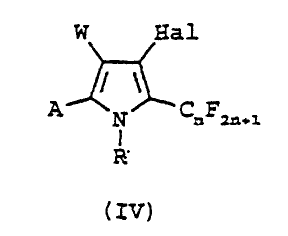 dans laquelle
R est l'hydrogène ou un groupe alkyle en C₁-C₆ facultativement substitué par un groupe alcoxy en C₁-C₄ ou phényle ;
n est un nombre entier 1, 2, 3, 4, 5, 6, 7 ou 8 ;
W est CN, NO₂, COOR₁ ou COR₂ ; 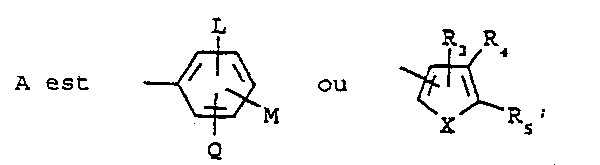
L est l'hydrogène ou un halogène ;
M et Q sont chacun indépendamment l'hydrogène, un halogène, CN, NO₂, un groupe alkyle en C₁-C₄, halogénoalkyle en C₁-C₄, alcoxy en C₁-C₄, halogénoalcoxy en C₁-C₄, alkylthio en C₁-C₄, alkylsulfinyle en C₁-C₄, ou bien, lorsque M et Q sont sur des positions adjacentes, ils peuvent être pris avec les atomes de carbone auxquels ils sont fixés pour former un cycle dans lequel MQ représente la structure
-OCH₂O-, -OCF₂O- ou -CH=CH-CH=CH- ;
R₁ et R₂ sont chacun indépendamment un groupe alkyle en C₁ - C₄ ;
R₃, R₄ et R₅ sont chacun indépendamment l'hydrogène, un halogène, NO₂, CHO, ou bien R₄ et R₅ peuvent être pris avec les atomes auxquels ils sont fixés pour former un cycle dans lequel R₄R₅ représente la structure 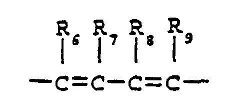
R₆, R₇, R₈ et R₉ sont chacun indépendamment l'hydrogène, un halogène, CN ou NO₂ ;
X est O ou S ; et
Hal est un atome d'halogène ;
qui consiste à faire réagir un amide-nitrile de formule II 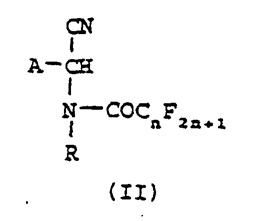 dans laquelle A, R et n sont tels que définis ci-dessus pour la formule I, avec au moins un équivalent molaire d'un composé diénophile de formule III 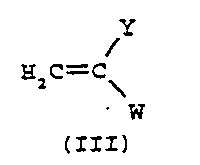 dans laquelle w est tel que défini ci-dessus pour la formule I et Y est l'hydrogène, Br ou Cl, à condition que si R est l'hydrogène, alors Y soit Br ou Cl, en présence d'un acide et d'un solvant et essentiellement en l'absence d'eau, pour former un composé de formule I 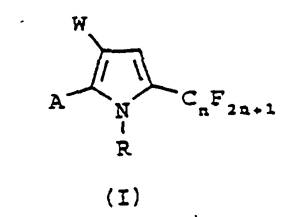 et à halogéner ledit composé de formule I pour former ledit composé de formule IV.

7. Composé de formule IIa 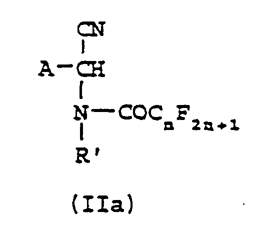 dans laquelle
n est un nombre entier 1, 2, 3, 4, 5, 6, 7 ou 8 ;
R' est un groupe alkyle en C₁-C₆ facultativement substitué par un groupe alcoxy en C₁-C₄ ou phényle ; 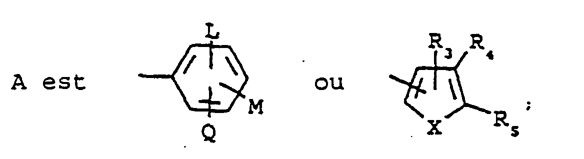
L est l'hydrogène ou un halogène ;
M et Q sont chacun indépendamment l'hydrogène, un halogène, CN, NO₂, un groupe alkyle en C₁-C₄, halogénoalkyle en C₁-C₄, alcoxy en C₁-C₄, halogénoalcoxy en C₁-C₄, alkylthio en C₁-C₄, alkylsulfinyle en C₁-C₄, ou bien, lorsque M et Q sont sur des positions adjacentes, ils peuvent être pris avec les atomes de carbone auxquels ils sont fixés pour former un cycle dans lequel MQ représente la structure
-OCH₂O-, -OCF₂O- ou -CH=CH-CH=CH- ;
R₁ et R₂ sont chacun indépendamment un groupe alkyle en C₁-C₄ ;
R₃, R₄ et R₅ sont chacun indépendamment l'hydrogène, un halogène, NO₂, CHO, ou bien R₄ et R₅ peuvent être pris avec les atomes auxquels ils sont fixés pour former un cycle dans lequel R₄R₅ représente la structure 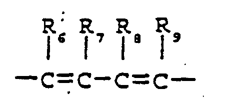
R₆, R₇, R₈ et R₉ sont chacun indépendamment l'hydrogène, un halogène, CN ou NO₂ ; et
X est O ou S.

8. Composé selon la revendication 7, dans lequel n est 1 ou 2.

9. Composé selon la revendication 7, dans lequel R' est un groupe méthyle ou éthoxyméthyle.

10. Composé selon la revendication 7, dans lequel A est 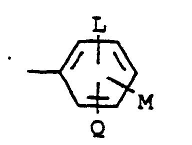 où L est l'hydrogène ou un halogène, et M et Q sont chacun indépendamment l'hydrogène, un halogène ou un groupe halogénoalkyle en C₁-C₄.
